# EUROPÄISCHE PATENTSCHRIFT

(11) **EP 0 506 776 B1**
(45) Veröffentlichungstag und Bekanntmachung des Hinweises auf die Patenterteilung: **14.09.1994**
(21) Anmeldenummer: 91901525.5
(22) Anmeldetag: 13.12.1990
(51) Int. Cl.: C07D 307/92

(54) **VERFAHREN ZUR HERSTELLUNG VON SCLAREOLID**
PROCESS FOR PRODUCING SCLAREOLIDE
PROCEDE POUR LA FABRICATION DE SCLAREOLURE

(30) Priorität: 21.12.1989 DE 3942358
(43) Veröffentlichungstag der Anmeldung: 07.10.1992
(73) Patentinhaber: Henkel Kommanditgesellschaft auf Aktien, 40191 Düsseldorf (DE)
(72) Erfinder: GERKE, Thomas, Dr., D-4040 Neuss 1 (DE); BRUNS, Klaus, Prof., D-4150 Krefeld-Traar (DE)
(86) Internationale Anmeldenummer: EP9002166
(87) Internationale Veröffentlichungsnummer: WO9109852

(56) Entgegenhaltungen:
- US-A- 3 050 532

## Beschreibung

Die Erfindung betrifft ein Verfahren zur Herstellung von Sclareolid aus Sclareol.

8 α, 12-Oxido-13,14,15,16-tetranorlabdan (Ambroxan^{R}) ist ein außerordentlich wertvoller Ambrariechstoff, der in der Ambra, einer Stoffwechselausscheidung des Pottwals, enthalten ist (Ullmann's Encyklopädie der Technischen Chemie, Band 20, Seite 283, Verlag Chemie Weinheim 1981). Synthetisch kann Ambroxan^{R} aus Sclareol durch zweistufige Oxidation gemäß US 30 50 532 und nachfolgende Reduktion des gebildeten Sclareolids hergestellt werden. Während des zweistufigen Oxidationsprozesses wird Sclareol (1) zunächst mit Kaliumpermanganat unter alkalischen Reaktionsbedingungen zu dem Hydroxyketon (2) oxidiert. Das gebildete Hydroxyketon wird ohne Isolierung mit Eisessig in den Enolether (3) umgewandelt und anschließend entweder mit Kaliumpermanganat oder mit Chromsäure oxidiert. Das erhaltene Oxidationsprodukt, ein Gemisch aus Sclareolid und Acetoxysäure, wird verseift und vollständig zu Sclareolid (4) cyclisiert. Nachteil dieses Verfahrens ist jedoch die lange Reaktionsdauer der Zweiten Oxidationsstufe. Gemäß den Beispielen der US 30 50 532 liegt sie bei etwa 19,5 Stunden. 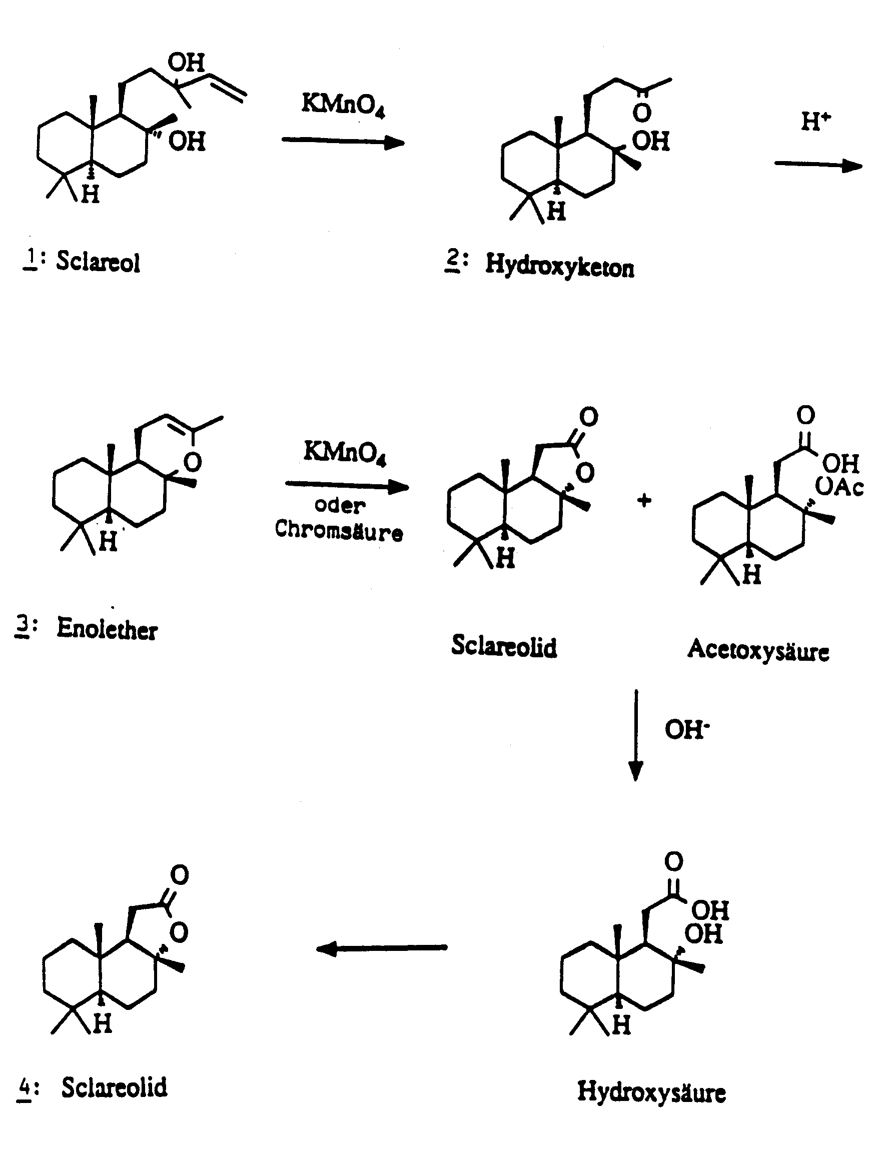
Aus DE 36 10 063 ist ferner bekannt, Sclareol mit Wasserstoffperoxid in Gegenwart von Säuren, beispielsweise p-Toluolsulfonsäure zu einem Hydroperoxid zu oxidieren, aus dem mit einem Redoxsystem, beispielsweise FeSO₄/Cu(OAc)₂, Ambroxan^{R} hergestellt werden kann. Auch bei diesem Verfahren muß für die Oxidationsstufe eine lange Reaktionszeit, nämlich 7 Tage, in Kauf genommen werden.

Die der Erfindung zugrunde liegende Aufgabe bestand daher in der Entwicklung eines Verfahrens zur Herstellung von Sclareolid, das keine langen Reaktionszeiten erfordert. Die Verkürzung der Reaktionsdauer soll mit mindestens ebenso guten Ausbeuten an Sclareolid, wie sie mit dem aus US 30 50 532 bekannten Verfahren erzielt werden können, verbunden sein.

Überraschenderweise wurde gefunden, daß die Reaktionszeiten erheblich reduziert werden können, wenn Sclareol in erster Stufe entweder mit Hypochloritsalzen in Gegenwart von Rutheniumverbindungen oder analog US 30 50 532 mit Kaliumpermanganat oxidativ abgebaut wird und in zweiter Stufe das erhaltene Hydroxyketon mit Persäuren und/oder deren Salzen zu Sclareolid oxidiert wird.

Gegenstand der Erfindung ist dementsprechend ein zweistufiges Verfahren zur Herstellung von Sclareolid aus Sclareol, welches dadurch gekennzeichnet ist, daß man Sclareol in erster Stufe entweder mit Hypochloritsalzen in Gegenwart von Rutheniumverbindungen oder in an sich bekannter Weise mit Kaliumpermanganat oxidativ zum Hydroxyketon 2 und/oder Enolether 3 abbaut und in zweiter Stufe Hydroxyketon 2 und/oder Enolether 3 mit Persäuren und/oder Persäuresalzen zum Sclareolid oxidiert.

Nach dem erfindungsgemäßen Verfahren kann Sclareol mit Alkali- und/oder Erdalkalihypochloriten, beispielsweise Natrium- und/oder Calciumhypochlorit, in Gegenwart von Rutheniumsalzen, beispielsweise Rutheniumdioxid und/oder Rutheniumtrichlorid, bei Temperaturen zwischen 0 und 40 °C oxidativ zum Hydroxyketon 2 und/oder Enolether 3 abgebaut werden. Es kann zweckmäßig sein, die Reaktion in Gegenwart von Phasentransferkatalysatoren, beispielsweise Ammoniumverbindungen, wie Tetrabutylammoniumchlorid durchzuführen. Sclareol wird vorzugsweise mit 0,1 bis 10 Mol-%, besonders bevorzugt mit 0,5 bis 3 Mol-% Rutheniumverbindungen und vorzugsweise mit 0 bis 20 Mol-%, besonders bevorzugt mit 0 bis 10 Mol-% Phasentransferkatalysatoren - alle Mol-%-Angaben beziehen sich auf Sclareol - in, unter den Reaktionsbedingungen inerten Lösungsmitteln, beispielsweise Methylenchlorid und/oder Cyclohexan, gelöst. Zu diesen Lösungen werden wäßrige Lösungen, die, bezogen auf die eingesetzte Menge an Sclareol, vorzugweise 400 bis 1000 Mol-%, besonders bevorzugt 600 bis 800 Mol-% Hypochloritsalze und vorzugsweise 0 bis 500 Mol-%, besonders bevorzugt 100 bis 500 Mol-% Alkalihydroxide, wie Natrium- und/oder Kaliumhydroxid, enthalten, getropft. Nach beendeter Reaktion kann das Reaktionsgemisch mit Säuren, zum Beispiel Ameisensäure, Essigsäure, Salzsäure und/oder Schwefelsäure, versetzt werden, wodurch aus dem gebildeten Hydroxyketon der entsprechende Enolether entsteht. Danach wird die organische Phase in an sich bekannter Weise von der wäßrigen Phase getrennt und aufgearbeitet und die Lösungsmittel destillativ entfernt.

Eine weitere Möglichkeit Sclareol oxidativ abzubauen, besteht darin, Sclareol mit Kaliumpermanganat analog US 30 50 532 zu versetzen. Hierbei wird Sclareol in Form einer wäßrigen Lösung mit - bezogen auf die eingesetzte Menge an Sclareol - vorzugsweise 300 bis 600 Mol-%, besonders bevorzugt 350 bis 550 Mol-% Kaliumpermanganat unter starkem Rühren bei Temperaturen zwischen 20 und 40 °C umgesetzt. Nach beendeter Reaktion wird zur Abtrennung von Braunstein das Reaktionsgemisch entweder mit Säuren, beispielsweise Schwefelsäure, auf einen pH-Wert von 2 eingestellt und anschließend bei 5 bis 10 °C SO₂ bis zur vollständingen Auflösung von Braunstein eingeleitet oder mit organischen Lösungsmitteln, wie Diethylether, Methylenchlorid oder Toluol, extrahiert.

Das nach einem der beiden obengenannten Verfahren hergestellte Hydroxyketon 2 und/oder der Enolether 3 - das Hydroxyketon kann vor der Zweiten Oxidationsstufe gewünschtenfalls mit Säuren, wie Ameisensäure, Essigsäure, Salzsäure und/oder Schwefelsäure, in den Enolether 3 überführt werden - werden mit Persäuren und/oder deren Salzen bei Temperaturen zwischen 10 und 60 °C zu Sclareolid oxidiert. Beispiele geeigneter Persäuren und/oder Persäuresalze sind Perameisensäure, die vorzugsweise in situ aus Ameisensäure und Wasserstoffperoxid hergestellt wird, Peressigsäure, Monoperoxyphthalsäure, Magnesiummonoperoxyphthalat und/oder m-Chlorperbenzoesäure. Bezogen auf die eingesetzte Menge an Hydroxyketon und/oder Enolether werden vorzugweise 100 bis 500 Mol-%, besonders bevorzugt 100 bis 300 Mol-% Persäuren und/oder Persäuresalze eingesetzt. Nach beendeter Oxidation wird mit Alkalien, beispielsweise Natronlauge und/oder methanolischer KOH verseift und die unverseifbaren Bestandteile in an sich bekannter Weise abgetrennt. Nach Ansäuern wird die gebildete Hydroxysäure abfiltriert und bei Temperaturen zwischen 130 und 160 °C zu Sclareolid cyclisiert.

Für die Herstellung von Sclareolid aus Sclareol nach dem erfindungsgemäßen Verfahren werden Reaktionszeiten benötigt, die erheblich unter denen der bekannten Verfahren liegen. Beispielsweise dauert die zweite Oxidationsstufe des erfindungsgemäßen Verfahrens etwa 3 Stunden, während die Reaktionszeit der zweiten Oxidationsstufe des aus US 30 50 532 bekannten Verfahrens - bei in der gleichen Größenordnung liegenden Einsatzmengen an Sclareol - etwa 19 bis 20 Stunden beträgt. Die Ausbeute an Sclareolid ist mit 65 bis 70 %, bezogen auf Sclareol, mindestens genauso hoch wie bei den aus dem Stand der Technik bekannten Verfahren.

### Beispiele

### Oxidativer Abbau von Sclareol

123,2 g Sclareol wurden mit 11,8 g Tetrabutylammoniumchlorid und 1,2 g RuO₂ in 500 ml Methylenchlorid gelöst. Zu dieser Mischung wurde innerhalb von 3 Stunden eine Lösung von 72 g Natriumhydroxid in 1530 g Natriumhypochlorit (12,6 gew.-%ig) getropft. Die Temperatur wurde durch Kühlen unter 25 °C erhalten.

Zur Aufarbeitung wurde das Reaktionsgemisch mit 37 gew.-%iger Salzsäure versetzt. Die wäßrige Phase wurde mit Methylenchlorid zweimal extrahiert, die vereinigten organischen Phasen mit Natriumsulfat getrocknet und das Lösungsmittel im Vakuum destillativ entfernt. Es wurden 93,4 g einer Mischung erhalten, die 89,3 % Enolether (gaschromatografische Bestimmung), entsprechend einer Ausbeute von 80 %, enthielt.

### Oxidation des Enolethers zum Sclareolid

93,4 g Enolether (89,3 %ig gemäß gaschromatografischer Bestimmung) wurden zu 1000 ml Ameisensäure gegeben. Dazu wurden 115 g Wasserstoffperoxid (30 gew.-%ig) getropft, wobei die Temperatur durch Kühlen unter 40 °C gehalten wurde. Nach dreistündigem Rühren wurde mit Diethylether extrahiert, mit Natriumsulfat getrocknet und anschließend der Ether destillativ entfernt. Der erhaltene Rückstand wurde in einer Lösung aus 66 g Kaliumhydroxid in 90 ml Wasser und 900 ml Methanol 3 Stunden unter Rückfluß gekocht. Anschließend wurde Methanol abdestilliert, der Rückstand in 2 l Wasser gelöst, mit 2,5 l Hexan extrahiert und die wäßrige Phase mit 20 gew.-%iger Schwefelsäure auf einen pH-Wert von 2 eingestellt. Die ausgefallene Hydroxysäure wurde abfiltriert und nach dem Trocknen auf 145 °C erhitzt, wobei sie unter Wasserabspaltung zu Sclareolid cyclisierte.

Nach der Umkristallisation aus Hexan erhielt man Sclareolid in einer Ausbeute von 65 %, bezogen auf Sclareol.

## Patentansprüche

1. Zweistufiges Verfahren zur Herstellung von Sclareolid aus Sclareol, dadurch gekennzeichnet, daß man Sclareol in erster Stufe entweder mit Hypochloritsalzen in Gegenwart von Rutheniumsalzen oder in an sich bekannter Weise mit Kaliumpermanganat oxidativ zum Hydroxyketon 2 und/oder Enolether 3 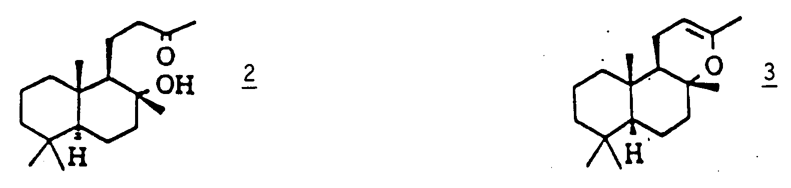 abbaut und in zweiter Stufe Hydroxyketon 2 und/oder Enolether 3 mit Persäuren und/oder Persäuresalzen zum Sclareolid oxidiert.

2. Verfahren nach Anspruch 1, dadurch gekennzeichnet, daß man Sclareol in erster Stufe entweder mit 400 bis 1000 Mol-%, vorzugweise mit 600 bis 800 Mol-% Alkali- und/oder Erdalkalihypochloritsalzen in Gegenwart von 0,1 bis 10 Mol-%, vorzugweise von 0,5 bis 3 Mol-% Rutheniumsalzen oder mit 300 bis 600 Mol-%, vorzugsweise 350 bis 550 Mol-% Kaliumpermanganat - alle Mol-%-Angaben bezogen auf Sclareol - oxidativ abbaut.

3. Verfahren nach einem oder beiden der Ansprüche 1 bis 2, dadurch gekennzeichnet, daß man den oxidativen Abbau von Sclareol mit Hypochloritsalzen in Gegenwart von 0 bis 20 Mol-%, vorzugweise von 0 bis 10 Mol-% Phasentransferkatalysatoren - alle Mol-%-Angaben bezogen auf Sclareol - durchführt.

4. Verfahren nach einem oder mehreren der Ansprüche 1 bis 3, dadurch gekennzeichnet, daß man in der zweiten Stufe 100 bis 500 Mol-%, vorzugsweise 100 bis 300 Mol-% Persäuren und/oder Persäuresalze - alle Mol-%-Angaben bezogen auf Hydroxyketon und/oder Enolether - einsetzt.

5. Verfahren nach einem oder mehreren der Ansprüche 1 bis 4, dadurch gekennzeichnet, daß man als Persäuren und/oder Persäuresalze Perameisensäure, Peressigsäure, Monoperoxyphthalsäure, m-Chlorperbenzoesäure und/oder Magnesiummonoperoxophthalat einsetzt.

## Claims

1. A two-stage process for the production of sclareolide from sclareol, in that, in a first stage, sclareol is oxidatively degraded to the hydroxyketone 2 and/or the enolether 3 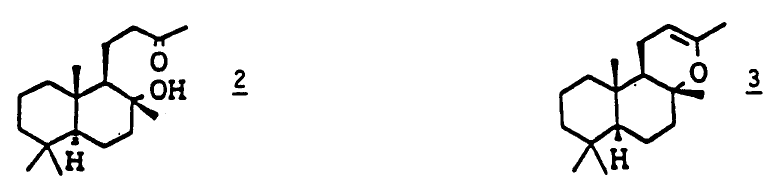 either with hypochlorite salts in the presence of ruthenium salts or in known manner with potassium permanganate and, in the second stage, the hydroxyketone 2 and/or the enolether 3 is oxidized to sclareolide with peracids and/or peracid salts.

2. A process as claimed in claim 1, characterized in that, in the first stage, sclareol is oxidatively degraded either with 400 to 1000 mol-% and preferably with 600 to 800 mol-% alkali metal and/or alkaline earth metal hypochlorite salts in the presence of 0.1 to 10 mol-% and preferably 0.5 to 3 mol-% ruthenium salts or with 300 to 600 mol-% and preferably 350 to 550 mol-% potassium permanganate (all mol-% based on sclareol).

3. A process as claimed in one or both of claims 1 and 2, characterized in that the oxidative degradation of sclareol is carried out with hypochlorite salts in the presence of 0 to 20 mol-% and preferably 0 to 10 mol-% phase transfer catalysts (all mol-% based on sclareol).

4. A process as claimed in one or more of claims 1 to 3, characterized in that 100 to 500 mol-% and preferably 100 to 300 mol-% peracids and/or peracid salts (all mol-% based on hydroxyketone and/or enolether) are used in the second stage.

5. A process as claimed in one or more claims 1 to 4, characterized in that performic acid, peracetic acid, monoperoxyphthalic acid, m-chloroperbenzoic acid and/or magnesium monoperoxophthalate are used as the peracids and/or peracid salts.

## Revendications

1. Procédé en deux étapes, de préparation du sclaréolure à partir du sclaréol, caractérisé en ce que l'on décompose le sclaréol dans une première étape soit avec des sels d'hypochlorite en présence de sels de ruthénium soit d'une manière connue en soi avec le permanganate de potassium, par oxydation en cétone hydroxylée 2 et/ou en éther énolique 3. 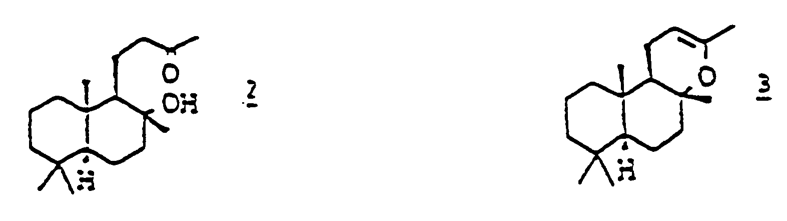 et que dans une deuxième étape, on oxyde la cétone hydroxylée 2 et/ou l'éther énolique 3 avec des péracides et/ou des sels de peracide, en sclaréolure.

2. Procédé selon la revendication 1, caractérisé en ce que l'on décompose par oxydation le sclaréol dans une première étape soit avec 400 à 1000 % molaire, de préférence avec 600 à 800 % molaire, de sels d'hypochlorite de métal alcalin et/ou de métal alcalino-terreux en présence de 0,1 à 10 % molaire, de préférence de 0,5 à 3 % molaire, de sels de ruthénium soit avec 300 à 600 % molaire, de préférence 350 à 550 % molaire, de permanganate de potassium, toutes les données en % molaire étant rapportées au sclaréol.

3. Procédé selon l'une ou les deux revendications 1 à 2, caractérisé en ce que l'on effectue la décomposition par oxydation du sclaréol avec des sels d'hypochlorite en présence de 0 & 20 % molaire, de préférence de 0 à 10 % molaire, de catalyseurs de transfert de phase, toutes les données en % molaire se rapportant au sclaréol.

4. Procédé selon l'une ou plusieurs des revendications 1 à 3, caractérisé en ce que dans la deuxième étape on met en oeuvre 100 à 500 % molaire, de préférence 100 à 300 % molaire de péracides et/ou de sels de péracide, toutes les données en % molaire se rapportant à la cétone hydroxylée et/ou à l'éther énolique.

5. Procédé selon l'une ou plusieurs des revendications 1 à 4, caractérisé en ce que l'on met en oeuvre comme péracides et/ou sels de péracide, l'acide performique, l'acide péracétique, l'acide monopéroxyphtalique, l'acide m-chloroperbenzoique et/ou le monopéroxyphtalate de magnésium.
